Europäisches Patentamt

**European Patent Office**

**Office européen des brevets**

(19)

(11) Numéro de publication: **0 044 770**
**B1**

(12)

# FASCICULE DE BREVET EUROPEEN

(45) Date de publication du fascicule du brevet:
**15.02.84**

(51) Int. Cl.³: **A 61 B 3/12,** A 61 B 3/02

(21) Numéro de dépôt: **81401107.8**

(22) Date de dépôt: **09.07.81**

(54) **Réfractomètre objectif assisté par microprocesseur.**

(30) Priorité: **21.07.80 FR 8016001**

(43) Date de publication de la demande:
**27.01.82 Bulletin 82/4**

(45) Mention de la délivrance du brevet:
**15.02.84 Bulletin 84/7**

(84) Etats contractants désignés:
**DE GB**

(56) Documents cités:
**DE - A - 2 925 951**
**DE - A - 2 951 897**
**FR - A - 2 204 805**
**FR - A - 2 228 461**
**FR - A - 2 372 617**
**US - A - 3 969 020**
**US - A - 4 181 407**
**US - A - 4 190 332**

**COMPUTER DESIGN, vol. 17, no. 4, avril 1978**
**Concord/Mass, US "Microcomputer Control Eye's**
**Focus and Measures Refractive Error", pages 64,68,72**

(73) Titulaire: **ETAT-FRANCAIS représenté par le DELEGUE**
**GENERAL POUR L'ARMEMENT, Bureau des Brevets et**
**Inventions de la Délégation Générale pour**
**l'Armement 14, rue Saint-Dominique, F-75997 Paris**
**Armées (FR)**

(72) Inventeur: **Simon, Jacques, 84, rue d'Alleray,**
**F-75015 Paris (FR)**
Inventeur: **Corno, Joel, 30, rue de Dourdan,**
**F-91470 Angervilliers (FR)**
Inventeur: **Bruneau, Didier, 27 résidence "La Cerisaie",**
**F-91120 Palaiseau (FR)**

La présente invention concerne les réfractomètres objectifs

On appelle optomètre objectif ou réfractomètre objectif tout instrument qui permet au praticien de déterminer l'amétropie du patient par examen d'un test projeté sur sa rétine.

Parmi les instruments de ce type, on va examiner essentiellement les appareils ou l'information est acquise visuellement par le praticien.

Certains de ces appareils permettent de procéder aux examens de tests projetés sur la rétine d'un patient.

Le pointé est réalisé lorsque le test projeté à travers l'œil du patient apparaît net au praticien au niveau de la rétine du patient.

Différents appareils ont été réalisés selon ce principe.

Parmi ces appareils on peut citer le réfractomètre oculaire de RODENSTOCK, le réfractomètre de THORNER-BUSCH et le réfractomètre d'ARNULF. ·

L'emploi des réfractomètres objectifs a été décrit dans un article de G. SOBREPERE qui fait partie des conférences de la clinique ophtalmologique de l'Hôtel-Dieu éditées par les laboratoires H. FAURE d'ANNONAY en 1970.

Il existe un deuxième type de réfractomètre mettant en jeu le principe de SCHEINER qui consiste à assurer la projection par deux zones distinctes de la pupille d'un test linéaire orthogonal aux deux zones pupillaires dont l'image est située au voisinage de la rétine.

Si l'image est située dans le plan de la rétine on observe l'image d'une fente lumineuse unique.

Si l'image est située en avant ou en arrière de la rétine, on observe deux fentes lumineuses distinctes.

Le dédoublement de ces images est directement proportionnel au défaut de convergence de l'œil considéré.

Le réfractomètre de FINCHAM est réalisé selon le principe précité.

Depuis quelques années, il existe des réfractomètres automatiques qui ne nécessitent la présence d'une assistante médicale que pour positionner le patient par rapport à l'instrument.

Cette opération étant réalisée, l'instrument effectue directement le pointé optique par mesure photoélectrique et délivre sur un ticket les informations correspondant aux caractèristiques des verres correcteurs à prescrire au patient.

Un appareil de ce type est décrit au brevet français n° 2 228 461. Selon la technique décrite dans ce brevet, on projette l'image d'une mire sur la rétine du patient par l'intermédiaire d'un système optique. L'image opthalmoscopique est analysée après une double traversée de l'œil par une mire de pas identique à la mire objet, et un photodétecteur délivre un signal électrique proportionnel au contraste de l'image. Ce signal sert à alimenter un moteur qui modifie la position du système optique de manière à obtenir le signal maximum pour la focalisation optimale.

L'exploration de l'astigmatisme est assurée en faisant tourner simultanément la mire objet et la mire d'analyse suivant six méridiens.

La source de lumière utilisée est une source de rayons infrarouges. La mesure sur un œil dure 30 secondes.

Cet appareil est en fait une transposition photoélectrique des réfractomètres fonctionnant par examen visuel d'un test projeté sur la rétine d'un patient.

Un autre appareil de type automatique est décrit au brevet français n° 2 204 805.

Cet appareil utilise le principe de SCHEINER.

Selon ce principe, un rayon lumineux est animé d'un mouvement rectiligne. Après réflexion sur la rétine, de l'oeil d'un patient, le rayon lumineux tombe sur un récepteur à quatre quadrants. La combinaison des signaux en provenance de chaque plage sensible donne une information sur la puissance sphérique et une information sur la direction de l'axe du cylindre. Les signaux obtenus agissent sur deux moteurs.

Le premier moteur fait tourner l'appareil axialement de telle façon que les signaux correspondant à la mesure de l'axe du cylindre s'annulent.

Le second moteur déplace le détecteur le long de l'axe optique du système afin de l'amener au foyer.

On remarquera qu'après la mise en place du sujet, le centrage est assuré automatiquement.

Il existe donc deux grandes catégories de réfractomètres.

Les réfractomètres objectifs visuels qui sont des appareils simples et qui nécessitent l'intervention du praticien.

Les réfractomètres objectifs automatiques qui ne font appel qu'à du personnel paramédical utilisant des récepteurs, éventuellement un mini-ordinateur ou des circuits électroniques analogiques et qui délivrent directement les informations relatives à la correction à apporter à l'oeil du patient.

Ces derniers appareils utilisent la lumière infrarouge qui présente l'intérêt pour le patient de ne pas être visible, mais dont l'inconvénient est de réaliser, en raison du chromatisme de l'œil, une mesure ne correspondant pas à l'emploi normal de celui-ci. ·

Il en résulte que la correction chromatique est difficile à réaliser à partir de ces mesures, car on ignore son évolution avec les différents sujets.

Par ailleurs, si les réfractomètres objectifs visuels nécessitent l'intervention du praticien qui doit notamment effectuer un certain nombre de manipulations et de calculs fastidieux, les réfractomètres objectifs automatiques présentent l'inconvénient contraire en ce sens que leur fonctionnement ne peut être influencé en aucune manière par le médecin qui voit par conséquent lui échapper toute initiative qu'il pourrait prendre en fonction d'un diagnostic préliminaire qu'il aurait établi sur son patient.

L'invention vise à remédier tant aux inconvénients des réfractomètres visuels qu'à ceux des réfractomètres automatiques en créant un appareil dans lequel le praticien puisse réaliser le pointé et observer directement la qualité de l'image rétinienne, alors que la mesure proprement dite est prise en charge par des moyens qui assurent la saisie rapide des informations, leur traitement et qui fournissent directement des informations relatives à la correction à apporter à l'oeil examiné.

L'invention a également pour objet un réfractomètre objectif permettant à un praticien d'effectuer visuellement les pointés optiques en projetant sur la rétine du patient l'image d'au moins une source lumineuse ponctuelle, ledit réfractomètre comportant un statif portant des objectifs fixes et des moyens faisant partie d'une voie inférieure de projection d'image et d'une voie supérieure d'observation d'image, ainsi qu'un ensemble mobile portant un oculaire et comprenant des moyens complétant lesdites voies supérieure et inférieure, caractérisé en ce qu'il comporte en outre des moyens électroniques de commande comprenant un microprocesseur associé à un capteur de déplacements linéaires de la partie mobile par rapport au statif et un capteur de déplacements angulaires des tests autour des axes optiques desdites voies inférieure et/ou supérieure ainsi que des moyens de transmission sous la commande du praticien des informations données par lesdits capteurs.

D'autres caractèristiques de l'invention apparaitront au cours de la description qui va suivre, faite en référence aux dessins annexés, donnés uniquement à titre d'exemple et sur lesquels:

— La Fig. 1 est une vue schématique de l'ensemble optique et mécanique du réfractomètre suivant l'invention;

— la Fig. 2 est un schéma synoptique de l'ensemble électronique et informatique destiné à commander l'appareil de l'invention;

— la Fig. 3 est une vue en élévation et en coupe de la partie principale du réfractomètre de la Fig. 1;

— la Fig. 4 est une vue en coupe de dessus de la partie de l'appareil représentée à la Fig. 3, suivant la ligne X–X' de celle-ci;

— la Fig. 5 est une coupe suivant la ligne 5–5 de la Fig. 3;

— les Fig. 6 et 7 sont des projections de deux aspects des tests linéaires obtenus avec l'agencement de la Fig. 5;

— la Fig. 8 est une variante simplifiée de l'agencement de la Fig. 5;

— la Fig. 9 est une vue d'une autre variante de l'agemcement de la Fig. 5 dans laquelle on utilise un test constitué par un trou unique situé sur l'axe optique de l'appareil;

— la Fig. 10 est une vue du test suivant la flèche G de la Fig. 9;

— la Fig. 11 est une vue de l'aspect des tests $T_1$ et $T_2$ suivant la flèche F de la Fig. 9;

— la Fig. 12 est une vue correspondant à celles des Fig. 5, 8 et 9 montrant un dispositif de vérification de la correction incorporé à l'appareil de l'invention; et

— la Fig. 13 est une vue de face des deux éléments du réfracteur de la Fig. 12 disposés côte à côte.

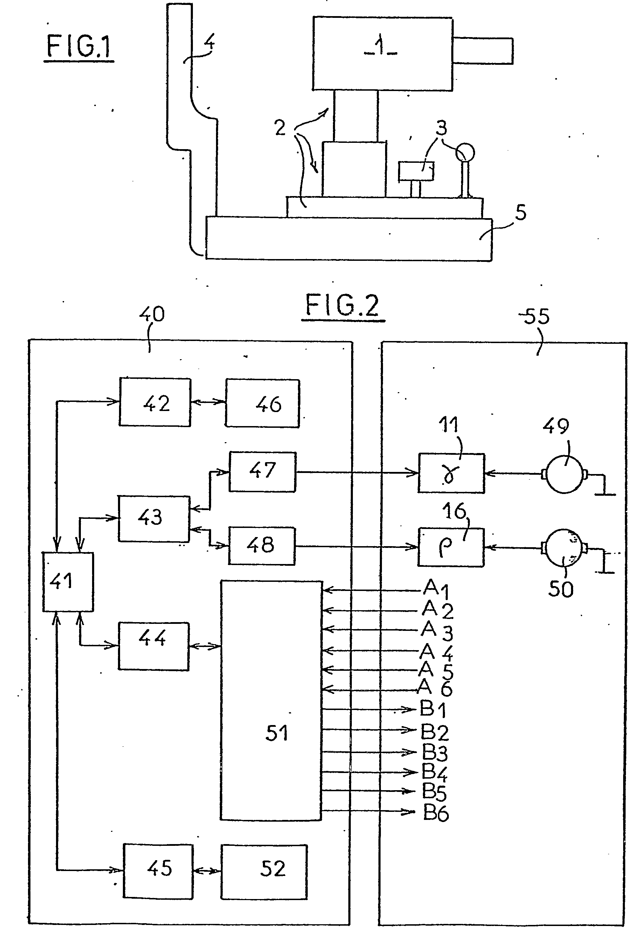

L'appareil suivant l'invention représenté schématiquement à la Fig. 1 comporte essentiellement une partie principale 1 du réfractomètre, montée sur un support 2 à trois degres de liberté muni de moyens de commande 3 appropriés, destinés à positionner l'appareil par rapport à l'œil du patient.

L'appareil comporte en outre un appuie-front 4 qui, avec le support 2, est fixé sur un socle 5.

Cet ensemble constitue le réfractomètre proprement dit et se pose sur une table.

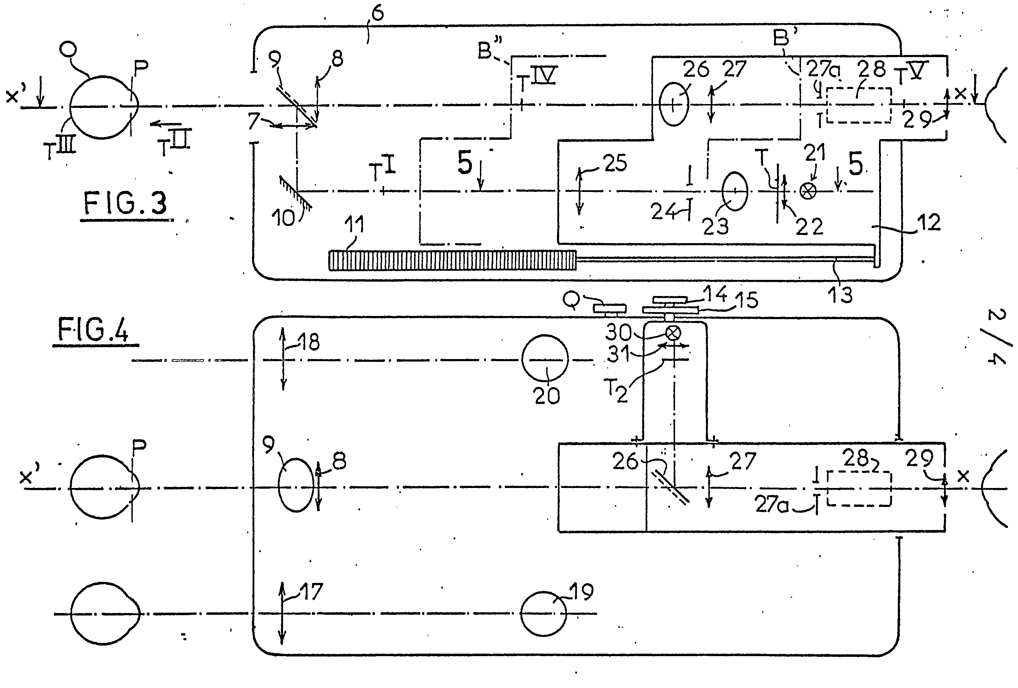

La partie principale 1 du réfractomètre est représentée plus en détail aux Fig. 3 et 4.

L'ensemble 1 comporte un statif 6, des objectifs 7, 8, identiques et à pupille télécentrique, dont les axes optiques sont perpendiculaires, un séparateur de faisceau 9 constitué par une lame semitransparente, disposée à l'intersection des axes optiques des objectifs 7 et 8, un miroir plan 10 placé sur l'axe optique de l'objectif 7 destiné à réfléchir les faisceaux lumineux qui traversent cet objectif dans une direction parallèle à l'axe optique de l'objectif 8. L'ensemble comporte en outre un capteur 11 de déplacements linéaires constitué par exemple par un potentiomètre dont le curseur est lié mécaniquement à un ensemble mobile 12 par l'intermédiaire d'une tige 13.

Ainsi qu'on peut le voir à la Fig. 4, le capteur 11 est actionnée par un bouton de commande 14 qui agit sur un pignon coopérant avec une crémaillère liée à la partie mobile 12 et qui n'est pas représentée sur les dessins.

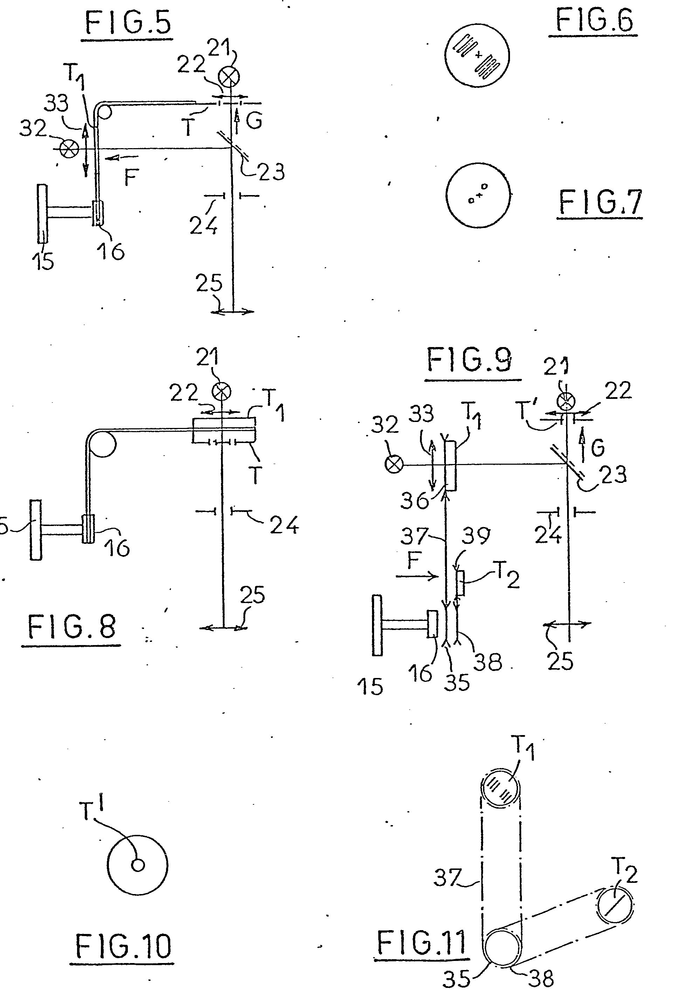

L'appareil comporte en outre un bouton de commande 15 destiné à assurer la rotation d'un capteur de rotation 16 (Fig. 8) et de deux tests T et T1 situés dans la partie mobile 12. Il comporte également des collimateurs 17 et 18 associés à des dispositifs d'éclairage 19 et 20 permettant d'obtenir un champ circulaire et uniforme.

L'ensemble mobile 12 est déplaçable en translation sur le statif 6 entre deux positions extrêmes B' et B".

Ainsi qu'on peut le voir à la Fig. 3, il comporte une voie inférieure et une voie supérieure.

Sur la voie inférieure, destinée à assurer la projection de l'image, on trouve une source lumineuse 21 qui peut être soit une lampe à filament de tungstène, soit une lampe à iode ou à arc, soit un laser à gaz ou solide, cette source étant destinée à éclairer par l'intermédiaire d'un condenseur 22 des trous T dont le diamètre est inférieur à 50 μm et qui constituent des sources auxiliaires parfaitement définies.

On trouve également sur la voie inférieure, un mélangeur de faisceau 23, une pupille 24 d'entrée du système, une lentille véhicule 25 destinée à assurer le transport des images T, le miroir 10, l'objectif 7 et le séparateur de faisceau 9, ces trois derniers éléments étant solidaires du statif 6.

La voie supérieure destinée à assurer l'observation de l'image comporte le séparateur 9 fonctionnant par transmission et l'objectif 8 solidaires du statif 6, l'objectif 8 étant identique à l'objectif 7, un mélangeur 26, un véhicule 27, un véhicule escamotable 28 et un oculaire 29.

Sur la Fig. 4, on retrouve les éléments 26, 27, 28 et 29. On voit en outre l'action du mélangeur de faisceau 26 qui permet de superposer au faisceau principal un graticule T2 éclairé par une source 30 et un condenseur 31 associés. Le graticule T2 constitue un repère d'accomodation et permet au praticien en agissant sur l'oculaire 29 de régler l'image du graticule T2 à sa convenance.

On retrouve également sur la Fig. 4, les collimateurs latéraux 17 et 18 associés aux dispositif d'éclairage 19 et 20. Sur cette Fig., on a représenté une mesure portant sur l'œil gauche du patient, de sorte que le dispositif d'éclairage 19 est allumé tandis que le dispositif d'éclairage 20 est éteint.

Sur la Fig. 5, on retrouve les éléments 21, 22, T, 23 et 24. On observe sur cette Fig. l'action du mélangeur de faisceau 23 qui permet de superposer aux trous T, les tests T1 éclairés par une source auxiliaire 32 à laquelle est associé un condenseur 33. La Fig. 5 montre également que le test T1 est lié en rotation avec le capteur angulaire 16 qui peut être actionné par le bouton de commande 15.

L'aspect en projection du test T1 est représenté à la Fig. 6.

L'aspect du test T en projection suivant la flèche G est représenté à la Fig. 7.

A la Fig. 8 on a représenté une variante simplifiée de l'agencement de la Fig. 5. Sur cette Fig. on retrouve les éléments 21, 22, T, T1, 24, 25, le capteur angulaire 16 et son bouton de commande 15. En revanche, les éléments 23, 32 et 33 de la Fig. 5 n'existent plus dans le mode de réalisation de la Fig. 8.

Une telle variante peut être réalisée en remplaçant le test T constitué dans le mode de réalisation de la Fig. 5 par un support opaque percé de trous, par un support présentant une faible transmission et constitué de trous à forte transmission. Cette réalisation est possible en utilisant les techniques de métallisation sous vide d'une lame de verre.

L'agencement de la Fig. 8 est avantageux en ce qu'il permet de supprimer une source auxiliaire et un mélangeur de faisceau.

Il est également possible de remplacer le test T constitué par deux ou plusieurs trous alignés, par un test T' constitué d'un seul trou situé sur l'axe optique de la voie inférieure. Bien entendu, dans un tel cas, il n'est pas nécessaire de faire tourner le test T', car une telle rotation ne produirait aucun effet. Un tel agencement est représenté à la Fig. 9 sur laquelle les éléments correspondant au mode de réalisation de la Fig. 5 sont désignés par les mêmes numéros de référence. Le bouton 15 de commande du capteur angulaire 16 assure également par l'intermédiaire de deux poulies 35 et 36 et d'une courroie 37, la rotation du test T1. Mais il assure également par l'intermédiaire d'un poulie 38 calée sur l'axe du bouton de commande 15 et d'une poulie 39, la rotation du test T2 de la voie supérieure constitué par un repère directionnel et éclairé par la source 30 (Fig. 4).

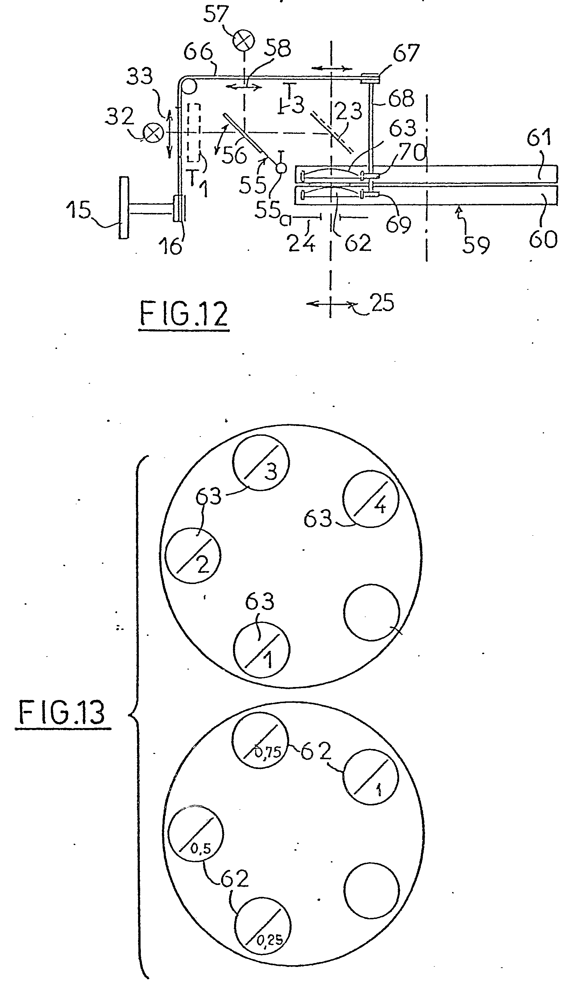

L'aspect du test T' est représenté à la Fig. 10 tandis que celui des tests T1 et T2 que l'on voit couplés en rotation par l'intermédiaire des poulies 35 et 38 est représenté à la Fig. 11.

L'ensemble électronique et informatique du réfractomètre suivant l'invention est représenté sous forme de schéma synoptique à la Fig. 2. Cet ensemble est divisé en deux parties:

Une première partie 40 comprend tous les éléments électroniques correspondant au traitement du signal. Elle comporte un microprocesseur 41 qui dans le présent exemple est un microprocesseur M6800 fabriqué et vendu par la Société MOTOROLA.

Le microprocesseur est connecté à quatre interfaces programmables 42 à 45. L'interface 42 est connectée à un dispositif d'affichage électronique 46.

L'interface 43 est connectée à des convertisseurs analogique-numérique 47 et 48 qui sont à leur tour connectés au capteur de déplacement linéaire $\gamma$ 11 et au capteur de rotation $\rho$ 16. Dans le mode de réalisation représenté à la Fig. 2, les capteurs 11 et 16 sont respectivement couplés à deux moteurs d'entraînement 49 et 50.

Le moteur 49 est destiné à entraîner l'ensemble mobile 12 vers une position d'initialisation après la fin de chaque mesure.

Le moteur 50 est destiné à faire tourner les tests $T_1$ et $T_2$ lors d'une mesure d'astigmatisme.

L'interface programmable 44 commande et reçoit les informations d'une interface 51 qui traite six informations d'entrée et qui délivre six informations de sortie. Les informations d'entrée sont les suivantes:

A1 indique que le réfractomètre est en position de réglage;

A2 indique que l'on examine l'œil droit (OD) ou l'œil gauche (OG);

A3 indique que l'on a mis en place un verre additionnel de puissance égale à − 7 dioptries;

A4 indique que l'on a mis en place un verre additionnel de puissance égale à + 7 dioptries;

A5 indique que l'on a appuyé sur le bouton poussoir Q d'entrée de données (Fig. 2);

A6 indique que l'on désire imprimer les résultats sur l'imprimante 52 connectée à l'interface programmable 45.

Les sorties de l'interface 51 sont les suivantes:

B1 assure la mise en marche ou l'arrêt du moteur 49 entraînant l'ensemble mobile 12 de la Fig. 3 vers sa position d'initialisation qui est une position hyperopique des patients;

B2 met en marche ou arrête le moteur 50 assurant l'entraînement en rotation des tests T et T1 ou des tests T2 et T1 pour un sujet astigmate suivant la disposition adoptée, ainsi qu'il résulte des Fig. 5 et 9;

B3 assure la signalisation par un voyant lumineux qu'un élément additionnel de puissance + 7 dioptries a été mis en place;

B4 indique à l'aide d'un voyant lumineux que l'on a mis en place un élément additionnel de puissance − 7 dioptries;

B5 détermine le sens de rotation du moteur qui entraîne l'ensemble mobile 12;

B6 indique le sens de rotation du moteur commandant la rotation des tests T, T1 ou T2, T1 suivant la disposition adoptée.

L'ensemble électronique représenté à la Fig. 2 comporte une deuxième partie indiquée par la référence générale 55 qui comprend, outre les capteurs de déplacement linéaire et angulaire 11 et 16 et les moteurs 49 et 50 qui leur sont associés, tous les éléments d'actionnement et de signalisation nécessaires à l'élaboration des entrées A1 á A6 de l'interface 51 et à l'exécution des instructions correspondant aux sorties B1 à B6 de cette interface.

Le fonctionnement du réfractomètre suivant l'invention va être tout d'abord examiné dans le cas d'une mesure sur un œil non astigmate.

On rappelle que l'œil non astigmate est un œil présentant une symétrie de révolution autour de son axe optique. Cet œil peut présenter un défaut de puissance.

La mise en place de l'appareil par rapport à la tête du sujet reposant sur l'appuie-front 4 représenté à la Fig. 1 est assurée par le praticien qui regarde dans l'oculaire 29 du réfractomètre, le véhicule 28 étant mis en place.

A cette situation correspond l'apparition d'un signal à l'entrée A1 de l'interface 51 du circuit de la Fig. 2. Cette information est transmise au microprocesseur 41 ce qui a pour effet de bloquer tout le processus de mesure.

Les seules informations prises en considération sont les informations A2 relatives à l'œil examiné, et à A3 et A4 relatives à la puissance des verres additionnels. Ces deux dernières informations parviennent au praticien sous la forme de signaux de sortie B3 et B4 qui provoquent l'alimentation de deux voyants lumineux non représentés. En position de réglage correspondant à la présence d'un signal sur l'entrée A1 de l'interface 51, les voyants $A_3$ ou $A_4$ clignotent. Cette opération étant effectuée, le véhicule 28 est escamoté et le praticien peut faire la mesure de l'amétropie sphérique du patient. L'information A1 disparait de l'entrée correspondante de l'interface et cette mesure est alors effectuée de la façon suivante. Le véhicule 25 de la voie inférieure de l'appareil assure le transport optique de la source auxiliaire T au point T1 situé sur l'axe optique de la voie inférieure. L'objectif 7, le miroir 10 et le séparateur 9 assurent la projection réelle ou virtuelle de la source $T^I$ en un point $T^{II}$ qui peut occuper n'importe quelle position appartenant à l'axe optique x–x' de la voie supérieure de l'appareil, dans un domaine dioptrique limité. Les différentes positions de $T^{II}$ sont obtenues en déplaçant l'ensemble mobile 12 par rapport au statif 6.

Le praticien agit sur le bouton de commande 14 représenté à la Fig. 4 afin de former l'image $T^{III}$ du point $T^{II}$ sur la rétine de l'oeil O du patient. Les objectifs 7 et 8 étant parfaitement identiques, le critère de formation de l'image est obtenu en cherchant à rendre ses dimensions minimales au travers de l'oculaire 29.

Si l'image $T^{III}$ réfléchie par la rétine est dans son plan, son image donnée par le système optique de l'œil se trouve à nouveau en $T^{II}$. Elle est alors reprise par le séparateur 9 qui agit par transmission et l'objectif 8 en donne une image qui se forme en $T^{IV}$. Le véhicule 27 donne de ce point une image située en $T^V$ observée par le praticien grâce à l'oculaire 29.

Les objectifs ophtalmoscopiques identiques 7 et 8 peuvent, dans certains cas, être des objectifs présentant pour le patient un tirage optique supérieur à leur focale.

Les verres additionnels de puissance ± 7 dioptries environ peuvent être placés, soit entre l'œil du patient et l'appareil, soit à l'intérieur de l'instrument dans les espaces conjugués du plan P de l'œil du patient, c'est-à-dire au voisinage de la pupille 24 de la voie inférieure et de la pupille 27a de la voie supérieure.

Les mesures effectuées par les capteurs 11 et 16 sont transmises à tout instant vers le microprocesseur 41 par l'intermédiaire des interfaces 47 et 48. Le microprocesseur effectue le calcul d'une fonction $Y = \dfrac{Ax + B}{Cx + D}$ où x est l'information délivrée par le capteur 11, tandis que A,B,C,D, sont des paramètres liés à l'optique employée dans le réfractomètre et sont choisis de manière que Y représente la valeur de la correction sphérique à placer à une distance donnée de 12 mm de sommet du la cornée de l'œil examiné.

Le microprocesseur 41 effectue également le calcul de l'azimut en degrés modulo 180° à l'aide de l'information délivrée par le capteur 16. Les informations de puissance dioptrique et d'azimut sont affichées en temps réel sur le dispositif d'affichage électronique 46. On remarquera que dans ce cas, l'information délivrée par le capteur 16 est transmise au microprocesseur mais n'est pas exploitée par celui-ci car il s'agit d'un œil non astigmate.

Lorsque le praticien a effectué le pointé, il appuie sur l'interrupteur Q ce qui envoie sur l'entrée A5 une information destinée au microprocesseur 41.

Le microprocesseur envoie au dispositif d'affichage 46 la valeur Y de la puissance sphérique qu'il a trouvée.

On va maintenant examiner le fonctionnement du réfractomètre suivant l'invention lors d'une mesure sur un oeil astigmate.

Le pointé unique qui dans l'exemple précédent donne la mesure de l'amétropie est remplacé par deux pointés obtenus par action sur le bouton 14 (Fig. 3) correspondant aux positions dioptriques des focales et par deux orientations angulaires obtenues par action sur le bouton 15 de déplacement angulaire, ces orientations correspondant aux directions des focales.

On réalise donc un premier pointé en transférant a l'aide du bouton poussoir Q, les informations provenant des capteurs 11 et 16 vers le microprocesseur 41. Après traitement il y a apparition de la valeur de la sphère sur le dispositif d'affichage électronique 46.

Le second pointé est ensuite effectué par le praticien à l'aide du bouton poussoir Q. Les nouvelles informations provenant des capteurs 11 et 16 sont de nouveau transmises au microprocesseur qui traite l'ensemble des quatre données et la nouvelle valeur de sphère Y qui correspond au second pointé; la valeur du cylindre obtenue par la différence des deux valeurs Y obtenues par chaque pointé ainsi que l'orientation angulaire sont affichées sur le dispositif 46.

Après le premier pointé, le moteur 50 (Fig. 2) fait tourner le bouton 15 auquel il est lié d'un angle de 90° sous l'action des signaux apparaissant sur les sorties B2 et B6 l'interface 51, ce qui évite le second pointé angulaire.

Dans tous les cas, que l'oeil soit non astigmate ou astigmate, le praticien peut obtenir sur un tikket délivré par l'imprimante 52, pour l'œil droit et l'œil gauche, les valeurs de la sphère, du cylindre, de son orientation angulaire. A cet effet, il suffit de provoquer à l'aide par exemple d'un interrupteur à bouton poussoir, l'apparition d'un signal sur l'entrée A6 l'interface 51.

Après impression l'ensemble mobile 12 est ramené sous l'action de signaux apparaissant sur les sorties B1 et B5 de l'interface 51 dans une position hypermétropique de consigne située à 5 dioptries à l'idde du moteur 49.

L'originalitè du réfractomètre qui vient d'être décrit vis à vis des réfractomètres classiques réside dans les points suivants:

Un microprocesseur est associé aux différents organes de déplacement d'éclairage et de mesure de l'appareil; On utilise des stratégies de mesure différentes suivant les programmes enregistrés dans les mémoires du microprocesseur, notamment en ce qui concerne la correction verre-œil;

Il y a retour automatique à la position hypermétropique avant un nouveau pointé;

La réfraction et l'azimut des focales sont affichés en temps réel en unités de puissance dioptrique et en degrés modulo 180 °C;

Les résultats obtenus pour chaque œil sont imprimés sur un ticket;

On emploie un test unidimensionnel constitué par un ou plusieurs lignes fines noires sur fond clair visible notamment sur la Fig. 6. Ce test peut tourner en synchronisme avec les trous T ou avec le test T2 de la voie supérieure suivant la disposition adoptée, ce qui permet au praticien de déterminer l'orientation de la focale astigmate; de plus ce test est décalé en position hypermétropique de l'ordre de 1 dioptrie afin de réaliser le principe correspondant à la méthode dite du brouillard.

Le réfractomètre assisté par microprocesseur qui vient d'être décrit se prête bien à une phase de vérification de la correction à apporter au sujet, cette fonction complémentaire pouvant être intégrée à l'appareil comme représenté aux Fig. 12 et 13.

La Fig. 12 est une vue correspondant à celle des Fig. 5, 8 et 9 de sorte que des numéros de référence identiques sont affectés aux éléments correspondants de ces figures. Mais l'appareil de la Fig. 12 comporte en outre un dispositif de vérification de la correction à apporter à l'œil dont on a déterminé l'amétropie.

Ce dispositif comporte un ensemble escamotable placé sur le trajet de la lumière émise par la source 32 éclairant le test T1.

L'ensemble 55 est constitué d'un miroir plan 56 et d'un test T3 portant un optotype solidaire dudit miroir et disposé par rapport à celui-ci de manière à être éclairé, par la lumière d'une source constituée par une lampe 57 et un condensateur 58 analogues à la lampe 32 et au condenseur 33, mais disposés de manière à pouvoir éclairer le test T3 par l'intermédiaire du miroir 56 lorsque celui-ci masque la lumière provenant de la lampe 32. L'ensemble 55 est monté à rotation autour d'un axe 55a.

Le dispositif de vérification comporte en outre un réfracteur 59 disposé dans l'espace conjugué de l'œil, entre la pupille 24 et le mélangeur de faisceau 23.

Ce réfracteur est de façon connue en soi formé de deux barillets coaxiaux 60, 61 montés à rotation autour d'un axe parallèle à l'axe optique de l'ensemble constitué par le mélangeur 23, la pupille 24 et la lentille véhicule 25.

Chaque barillet porte une série de lentilles cylindriques 62, 63 de puissances allant de 0,25 à 1 dioptrie pour le barillet 60 et de 1 à 4 dioptries pour le barillet 61.

Chaque barillet comporte en outre un trou sans lentille 64, 65.

Enfin, le dispositif comprote des moyens mécaniques de déplacement angulaire des lentilles 62 et 63 en synchronisme avec le test T1, ces moyens étant constitués par une courroie 66 actionnée par la poulie 16, liée au bouton 15 et passant sur une seconde poulie 67 solidaire d'un arbre 68 portant à son extrémité des pignons 69, 70 qui engrènent avec des couronnes non représentées, faisant partie des montures des lentilles 62, 63 correspondantes.

La correction sphérique est assurée par le déplacement de l'équipage mobile 12 par rapport au statif 6, la correction de l'astigmatisme est assurée par la mise en place d'une ou de deux lentilles cylindriques 62, 63 dans l'espace conjugué de l'œil du patient, c'est-à-dire à l'intérieur de l'appareil au voisinage du diaphragme 24 comme représenté à la Fig. 12. Dans ce cas, on maintient l'appareil dans la position du dernier pointé et l'on affiche sur un tambour (non représenté) la valeur dioptrique de la correction astigmate délivrée par la mesure. L'action de ce tambour revient à placer le ou les verres cylindriques correcteurs entre le diaphragme 24 et le mélangeur 23. Ce verre est mécaniquement pré-réglé en orientation par suite de la liaison angulaire linéaire entre l'orientation des tests (T1–T) on (T2–T1) et l'orientation du verre cylindrique correcteur.

Le réfracteur constitué de lentilles cylindriques tel que le réfracteur 59 est utilisé en optométrie subjective.

Au cours de la même séquence opératoire, on éteint la source 21 (Fig. 5 ou 9) et on remplace le test T1 par un test T3 constitué par la réduction microscopique d'un tableau d'optotype.

**Revendications**

1. Réfractomètre objectif permettant à un praticien d'effectuer visuellement des pointés en projetant sur la rétine du patient l'image d'au moins une source lumineuse ponctuelle, ce réfractomètre comportant un statif portant des objectifs fixes et des moyens faisant partie d'une voie inférieure de projection d'image et d'une voie supérieure d'observation d'image ainsi qu'un ensemble mobile portant un oculaire et comprenant des moyens complétant les voies supérieure et inférieure, caractérisé en ce qu'il comporte en outre des moyens électroniques de commande comprenant un microprocesseur (41) associé à un capteur (11) de déplacements linéaires de la partie mobile (12) par rapport au statif (6) et un capteur (16) de déplacements angulaires des tests (T1–T ou T1–T') autour des axes optiques des voies inférieure et/ou supérieure ainsi que des moyens (43, 47, 48) de transmission, sous la commande du praticien, des informations données par les capteurs.

2. Réfractomètre objectif suivant la revendication 1, caractérisé en ce que le microprocesseur (41) est connecté aux capteurs de déplacements linéaires (11) et de déplacements angulaires (16) par l'intermédiaire d'un circuit d'interface programmable (43) et de convertisseur analogique-numérique (47, 48) qui constituent avec le circuit d'interface les moyens de transmission.

3. Réfractomètre objectif suivant l'une quelconque des revendications 1 et 2, caractérisé en ce que le microprocesseur (41) est en outre connecté par l'intermédiation d'un circuit d'interface programmable (42) à un dispositif d'affichage numérique (46) de type connu.

4. Réfractomètre objectuf suivant l'une quelconque des revendications 1 à 3, caractérisé en ce que le microprocesseur (41) est en outre connecté par l'intermédiaire d'un circuit d'interface programmable (45) à une imprimante (52).

5. Réfractomètre objectif suivant l'une quelconque des revendications 1 à 4, caractérisé en ce qu'il comporte en outre un circuit d'interface (51) de traitement d'informations relatives aux manipulations effectuées par le praticien sur la partie optique du réfractomètre et de production de signaux de commande ou de signalisation d'opérations correspondant aux manipulations du praticien.

6. Réfractomètre objectif suivant l'une quelconque des revendications 1 à 5, caractérisé en ce qu'il comporte en outre des moyens (49) commandés par le microprocesseur (41) pour ramener automatiquement l'ensemble mobile (12) dans une position d'initialisation qui est une position hyperopique pour la majorité des patients, les moyens (49) étant constituées par un moteur électrique associé au capteur (11) de déplacements linéaires de la partie mobile (12) et étant commandé par le microprocesseur (41) par l'intermédiaire de l'interface (51), de l'interface programmable (43) et du convertisseur analogique numérique (47).

7. Réfractomètre objectif suivant l'une quelconque des revendications 1 à 6, pour l'examen d'un oeil astigmate, comportant en outre des moyens (50) pour faire tourner les tests (TI–T ou T1–T') d'un angle de 90° par rapport à l'œil du patient, ces moyens (50) étant constitués par un moteur électrique associé au capteur (16) de déplacements angulaires des tests (T1–T ou T1–T'), caractérisé en ce que le moteur est commandé par le microprocesseur (41) par l'intermédiaire de l'interface (51), de l'interface programmable (43) et du convertisseur analogique-numérique (48).

8. Réfractomètre objectif suivant l'une quelconque des revendications 1 à 7, caractérisé en ce qu'il comporte en outre un dispositif de vérification de la correction à apporter à l'oeil du patient, constitué par un réfracteur constitué de lentilles cylindriques (59) placé dans l'espace réel de l'œil ou dans un espace conjugué de celui-ci, les éléments correcteurs (62, 63) du réfracteur étant liés aux tests (T1, T ou T2, T') par des moyens (66 à 70) de déplacement en rotation en synchronisme des éléments correcteurs ed des tests, le dispositif comportant en outre un tableau d'optotypes (T3) escamotable pouvant être substitué aux tests (T1, T ou T2, T').

**Patentansprüche**

1. Objektivrefraktometer, mit dem der Augenarzt visuell optische Punkte untersuchen kann, indem er das Bild von mindestens einer Punktlichtquelle auf die Hornhaut des Patienten projiziert, wobei dieses Objektivrefraktometer ein Stativ umfasst, das feststehende Objektive trägt und Mittel, die zu einem unteren Bildprojektionsweg und zu einem oberen Bildbeobachtungsweg gehören, sowie eine mobile Baugruppe, die ein Okular trägt und die Mittel zur Vervollständigung der oberen und unteren Wege aufweist, dadurch gekennzeichnet, dass es zusätzlich elektronische Steuerorgane umfasst, zu denen ein Mikroprozessor (41) zusammen mit einer Sonde (11) für die lineare Verschiebung des mobilen Teils (12) gegenüber dem Stativ (6) und eine Sonde (16) für die Winkelverschiebungen der Tests (T1–T oder T1–T') um die optischen Achsen der unteren und/oder oberen Wege gehören, sowie vom Augenarzt bediente Übertragungsmittel (43, 47, 48) für die von den Sonden erfassten Informationen.

2. Objektivrefraktometer gemäss Anspruch 1, dadurch gekennzeichnet, dass der Mikroprozessor (41) an die Sonden für die linearen Verschiebungen (11) und die Winkelverschiebungen (16) angeschlossen ist, und zwar über einen programmierbaren Interfacekreis (43) und einen Analog-Digital-Wandler (47, 48), die mit dem Interfacekreis die Übertragungsmittel bilden.

3. Objektivrefraktometer gemäss einem der Ansprüche 1 und 2, dadurch gekennzeichnet, dass der Mikroprozessor (41) ausserdem über einen programmierbaren Interfacekreis (42) an eine numerische Anzeigevorrichtung (46) bekannten Typs angeschlossen ist.

4. Objektivrefraktometer gemäss einem der Ansprüche 1 bis 3, dadurch gekennzeichnet, dass der Mikroprozessor (41) ausserdem über einen programmierbaren Interfacekreis (45) an einen Drucker (52) angeschlossen ist.

5. Objektivrefraktometer gemäss einem der Ansprüche 1 bis 4, dadurch gekennzeichnet, dass es ausserdem einen Interfacekreis (51) zur Informationsverarbeitung bezüglich der vom Augenarzt am optischen Teil des Refraktometers vorgenommenen Bedienungen aufweist, und zur Erzeugung von Steuersignalen oder Bedienungsanzeigen gemäss den Bedienungen des Augenarztes.

6. Objektivrefraktometer gemäss einem der Ansprüche 1 bis 5, dadurch gekennzeichnet, dass es ausserdem durch den Mikroprozessor (41) gesteuerte Mittel (49) aufweist, mit denen automatisch der mobile Teil (12) in die Initialisierungsposition gebracht werden kann, die für die meisten Patienten eine hyperoptische Position ist, wobei die Mittel (49) aus einem elektrischen Motor in Verbindung mit der Sonde (11) für die linearen Verschiebungen des mobilen Teils (12) bestehen und Mikroprozessor (41) über das Interface (51), das programmierbare Interface (43) und den Analog-Digital-Wandler (47) gesteuert werden.

7. Objektivrefraktometer gemäss einem der Ansprüche 1 bis 6, dadurch gekennzeichnet, dass es für die Untersuchung eines astigmatischen Auges zusätzlich Mittel (50) umfasst, mit denen die Tests (TI–T oder TI–T') um einen 90°-Winkel im Vergleich zum Auge des Patienten gedreht werden können, wobei diese Mittel aus einem elektrischen Motor in Verbindung mit der Sonde (16) für Winkelverschiebungen der Tests (T1–T oder T1–T') bestehen, dadurch gekennzeichnet, dass der Motor vom Mikroprozessor (41) über das Interface (51), das programmierbare Interface (43) und den Analog-Digital-Wandler (48) gesteuert wird.

8. Objektivrefraktometer gemäss einem der Ansprüche 1 bis 7, dadurch gekennzeichnet, dass es ausserdem eine Vorrichtung für die Überprüfung der dem Auge des Patienten angemessenen Korrektur umfasst, bestehend aus einem Refraktor, der aus zylindrischen Linsen (59) gebildet wird und in den wirklichen Augenraum oder einen davon abgeleiteten Raum eingebracht wird, die Korrekturelemente (62, 63) des Refraktors stehen mit den Tests (T1, T oder T2, T') in Verbindung über Mittel (66 bis 70) der Verschiebung in Rotation mit Synchronismus der Korrekturelemente und der Tests, wobei die Vorrichtung ausserdem eine schwenkbare Tafel mit Optotypen (T3) umfasst, die an die Stelle der Tests (T1, T oder T2, T') treten kann.

## Claims

1. An objective refractometer which permits a practitioner visually to effect optical pointings by projecting onto the retina of a patient the image of a least one point light source, said refractometer comprising a stand bearing stationary objectives and means forming part of a lower image projection path and of an upper image observation path as well as a movable unit bearing an eyepiece and comprising means supplementing the said upper and lower paths, characterized by the fact that it furthermore comprises electronic control means comprising a microprocessor (41) associated with a detector (11) of linear displacements of the movable part (12) with respect to the stand (6) and a detector (16) of angular displacements of the test patterns (T1–T) or (T1–T') around optical axes of said lower and/or upper paths as well as means (43, 47, 48) for transmission, under the control of the practitioner, of the information given by said detectors.

2. An objective refractometer according to Claim 1, characterized by the fact that said microprocessor (41) is connected to said linear displacement detector (11) and angular displacement detector (16) via a programmable interface circuit (43) and a analog-digital converter (47, 48) which constitute said transmission means with said interface circuit.

3. An objective refractometer according to either of Claims 1 and 2, characterized by the fact that said microprocessor (41) is furthermors operatively connected by a programmable interface circuit (42) to a digital display device (46).

4. An objective refractometer according to any of Claims 1 to 3, characterized by the fact that the microprocessor (41) is furthermore operatwely connected to a printer (52) by a programmable interface circuit (45).

5. An objective refractometer according to any of Claims 1 to 4, characterized by the fact that it comprises furthermore an interface circuit (51) for the processing of information relative to manipulations effected by the practitionner on the optical part of the refractometer and for production of control signals or operation signals corresponding to the manipulations of the practitionner.

6. An objective refractometer according to any of Claims 1 to 5, characterized by the fact that it furthermore comprises means (49) driven by the microprocessor (41) in order to return the movable unit (12) automatically back to an initialization position which is a hyperopic position for the majority of patients, the said means (50) being formed by an electric motor associated with the linear displacement detector (11) of the movable part (12) and being controlled by the microprocessor (41) via the interface (51), the programmable interface (43) and the analogdigital converter (47).

7. An objective refractometer according to any of Claims 1 to 6, characterized by the fact that in order to examine an astigmatic eye, it furthermore comprises means (50) for turning the test patterns (T1–T or T1–T' through an angle of 90° with respect to the eye of the patient, said means (50) being formed of an electric motor associated with the angular displacement detector (16) of the test patterns (T1–T or T1–T') and controlled by the microprocessor (41) via the interface (51), the programmable interface (43) and the analog-digital converter (48).

8. An objective refractometer according to any of Claims 1 to 7, characterized by the fact that it furthermore comprises a device for verifying the correction to be made in the eye of the patient, consisting of a refractor formed of cylindrical lenses (59) which is placed in the real space of the eye or in the conjugate space thereof, the correction element (62, 63) of the refractor being related to the tests (T1, T or T2, T') by means (66 to 70) for displacement in rotation in synchronism of the corrector elements and test patterns, the device furthermore comprising a retractable optotype table (T3) which can be substituted for the said test patterns (T1, T or T2, T').

0 044 770

FIG.1

FIG.2

FIG.3

FIG.4

FIG.5

FIG.6

FIG.7

FIG.8

FIG.9

FIG.10

FIG.11

FIG.12

FIG.13